# EUROPEAN PATENT SPECIFICATION

(11) **EP 1 863 558 B1**
(45) Date of publication and mention of the grant of the patent: **17.01.2018**
(21) Application number: 06721663.0
(22) Date of filing: 15.03.2006
(51) Int. Cl.: A61B 18/14, A61N 1/06, A61B 17/00, A61B 90/00

(54) **ELECTROSURGICAL CANNULA**
ELEKTROCHIRURGISCHE KANÜLE
CANULE ELECTROCHIRURGICALE

(30) Priority: 15.03.2005 US 79318; 22.08.2005 US 207707
(43) Date of publication of application: 12.12.2007
(73) Proprietor: Avent, Inc., Alpharetta, GA 30004 (US)
(72) Inventor: CHANDRAN, Subashini, Toronto, Ontario M6P 2P4 (CA); SHAH, Krishan, Mississauga, Ontario L5M 2C7 (CA); GODARA, Neil, Mississauga, Ontario L5L 3T8 (CA)
(74) Representative: Davies, Christopher Robert
(86) International application number: PCT/CA2006/000388
(87) International publication number: WO 2006/096978

(56) References cited:
- WO-A1-02/32500
- WO-A1-99/34860
- WO-A1-99/48548
- WO-A1-2004/020024
- WO-A1-2004/078052
- CA-A1- 1 160 932
- DE-A1- 10 228 085
- FR-A1- 2 854 052
- US-A- 4 657 024
- US-A1- 2004 106 891
- US-A1- 2004 176 759
- US-A1- 2004 267 203
- US-A1- 2005 049 570
- US-A1- 2005 049 570

## Description

### TECHNICAL FIELD

The invention relates to an electrosurgical device and more specifically to a device used in the treatment of pain through the application of energy.

### BACKGROUND OF THE ART

One limitation of the techniques used currently for radio frequency (RF) denervation is that the insulated shaft of the cannula is indistinguishable from the exposed distal tip of the cannula under X-ray fluoroscopy, due to the fact that the entire cannula, i.e. both the insulated as well as the exposed regions, is generally made up of a radiopaque substance. Therefore, precise localization of the conductive distal tip of the cannula is not possible as the entire cannula, comprising both the tip and the shaft, appears dark on the fluoroscopic image. Specific localization of the distal tip of the cannula is desirable as it is this region of the cannula that is electrically exposed and is therefore responsible for creating the lesion in the tissue.

The incorporation of a radiopaque marker onto other devices, for example catheters, for insertion into a patient's body, may result in a significant increase in the force required to insert the device into the patient's body, relative to the force required to insert a device lacking such a marker, due to the increased radial profile (or effective outer diameter) of the device. This increased force, in turn, may result in unnecessary damage to bodily tissue during insertion of such a device into a patient's body.

Furthermore, in many procedures, the target structure may lie substantially parallel to the cannula and, therefore, delivery of a treatment composition through only a distal aperture of the cannula may not produce the desired effect on the target structure. It would, therefore, be beneficial to provide a device that allows for more targeted delivery of treatment compositions to a structure positioned parallel to the cannula.

WO 2004/078052A1 discloses an electrical needle with radiopaque marker. WO 02/32500 discloses a cardiac transvenous defibrillation lead having a defibrillation electrode with a radiopaque marker. US 2005/0049570 discloses a transparent sheath for use in accessing the inside of a body with a radiopaque marking.

Thus, there is a need for an electrosurgical device that overcomes some or all of the limitations of the prior art.

### DISCLOSURE OF INVENTION

The present invention is directed to an improved cannula for insertion into a patient's body in accordance with claim 1, and a system for delivering energy to a patient's body in accordance with claim 10. As an example there is a method for treating pain using the cannula.

In accordance with the present invention, a cannula for insertion into a patient's body is provided. The cannula comprises an elongate member having a proximal region and a distal region and a lumen therebetween, and a radiopaque marker associated with the elongate member for identifying a portion of the elongate member using radiographic imaging techniques such as fluoroscopy. The radiopaque marker is shaped to reduce the force required to insert the cannula into the patient's body, by having a tapered end.

As a feature of the present invention, the elongate member is electrically conductive and comprises an electrically insulated region and an electrically exposed region. The radiopaque marker is associated with at least one of the electrically insulated region and the electrically exposed region for distinguishing the exposed region from the insulated region under radiographic imaging.

Also disclosed is a cannula for insertion into a patient's body. The cannula may comprise an elongate member having a distal region and a proximal region and defining a lumen therebetween and further comprising a wall defining at least one lateral aperture therethrough and a distal end defining at least one distal aperture. In addition, the distal region may comprise an electrically exposed and conductive distal tip and an outer surface of the cannula between the distal tip and the proximal region may be non-conductive.

Thus, the present invention describes a novel cannula, wherein the cannula optionally overcomes some or all of the deficiencies associated with the prior art. The cannula incorporates a radiopaque marker to allow for improved visualization of at least a portion of the cannula utilizing radiographic imaging techniques such as fluoroscopy. In addition, the radiopaque marker is structured to reduce the force required to insert the cannula comprising the radiopaque marker into a patient's body. Furthermore, the cannula may define at least one lateral aperture to allow for improved delivery of a treatment composition to a target site.

These features and others will become apparent in the detailed description that follows.

### BRIEF DESCRIPTION OF DRAWINGS

In order that the invention may be readily understood, embodiments of the invention are illustrated by way of examples in the accompanying drawings, in which:
Figure 1A is a top view of a radiopaque cannula with a radiopaque marker in accordance with the present invention;
Figure 1B is a sectional side view of the cannula depicted in Figure 1A;
Figures 2 to 4 are top views illustrating alternate arrangements of a radiopaque cannula with associated radiopaque markers not in accordance with the present invention;
Figure 5 is a top view illustrating an embodiment of a radiopaque cannula with associated radiopaque markers in accordance with the present invention;
Figure 6A is a top view of a radiopaque cannula not in accordance with the present invention comprising a rigidly bent distal portion and a lateral aperture;
Figure 6B is a sectional side view of the cannula depicted in Figure 6A;
Figure 7A is a top view of an alternate of the cannula shown in Figure 6A;
Figure 7B is a sectional side view of the cannula depicted in Figure 7A;
Figure 8A is a top view of a further cannula with a lateral aperture not in accordance with the present invention;
Figure 8B is a sectional side view of the cannula depicted in Figure 8 A;
Figures 9A-9C show top plan views, fragmented, of various cannulas not in accordance with the present invention having more than one lateral aperture;
Figure 10A is a top view of an embodiment of a radiopaque cannula of the present invention comprising a stylet;
Figure 10B is a top view of a stylet with a radiopaque marker;
Figure 11A is a top view of a cannula not in accordance with the present invention having a lateral aperture and further comprising a stylet;
Figure 11B is a top view of a stylet with a radiopaque marker as may be disposed within the cannula of Figure 11A;
Figure 12A is an isometric view of a stylet with a reduced mass region which may be used in accordance with the present invention;
Figure 12B is an isometric view of an embodiment of a radiopaque cannula which may be used in accordance with the present invention comprising a stylet with a reduced mass region;
Figures 13A - 13B show isometric views of an alternate embodiment of the cannula illustrated in Figures 12A - 12B;
Figure 14A is a schematic illustration of a radiopaque cannula comprising a radiopaque marker connected to a high frequency generator in accordance with the present invention; and
Figure 14B is a schematic illustration of an alternate cannula not in accordance with the invention comprising a lateral aperture, whereby the cannula is connected to a high frequency generator.

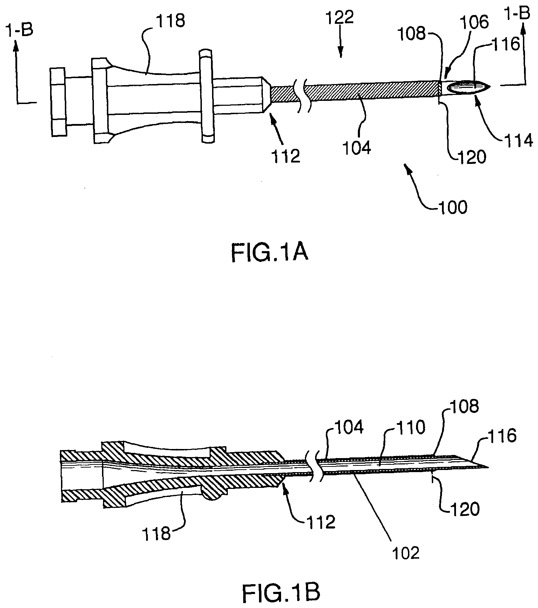

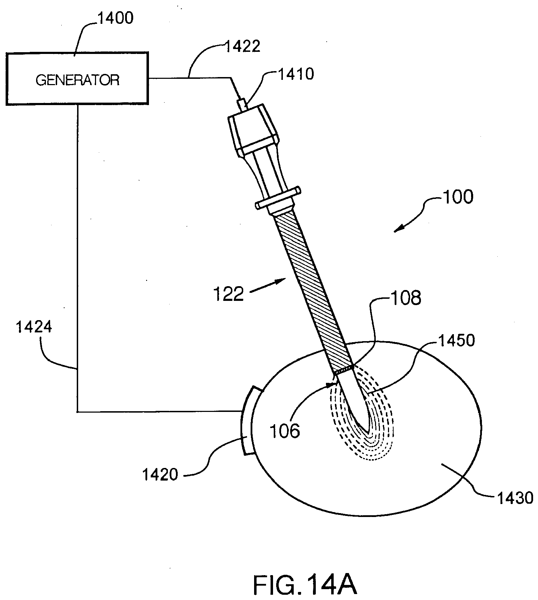

Cannulas in accordance with the invention are shown in Figures 1A, 1B, 5, 10A and in the system of Figure 14A. The remaining figures illustrate cannulas not in accordance with the invention but which have been retained to facilitate understanding of the invention, and features that may be used therewith, or illustrate stylets or a system which may be used with cannulas of the present invention.

### BEST MODES FOR CARRYING OUT THE INVENTION

With specific reference now to the drawings in detail, it is stressed that the particulars shown are by way of example and for purposes of illustrative discussion of certain embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

Prior to describing the drawings in detail, it should be noted that, throughout this description and corresponding drawings, like numerals are used to refer to like elements of the present invention.

### STRUCTURE

Referring first to Figures 1A and 1B, a first embodiment of an electrosurgical cannula 100 of the present invention is shown. Figure 1A shows a top view of cannula 100 while Figure 1B illustrates a sectional view of cannula 100 along line 1B- 1B in Figure 1A. In embodiments, cannula 100 is manufactured from an electrically conductive and radiopaque material, and an elongate member 102, for example a shaft 102, of cannula 100 is at least partially coated with an electrically insulating material 104. Suitable materials for electrically insulating material 104 include, but are not limited to, parylene and polytetrafluoroethylene (PTFE). A distal tip portion 106 of shaft 102 remains exposed and electrically conductive. Due to the conductive nature of distal tip portion 106, and for the purposes of this invention, distal tip portion 106 may be described alternatively as a distal tip, an active electrode or active tip. Distal tip portion 106 is optionally sharp to facilitate penetration into a patient's body. Alternatively, distal tip portion 106 may be blunt, rounded, straight, beveled, rigidly bent, or may take on other forms depending on the particular application. In the embodiment shown in Figure 1, a radiopaque band 108, described in greater detail below, is positioned so as to distinguish active tip 106 from insulated region 122 of shaft 102 under X-ray fluoroscopic imaging.

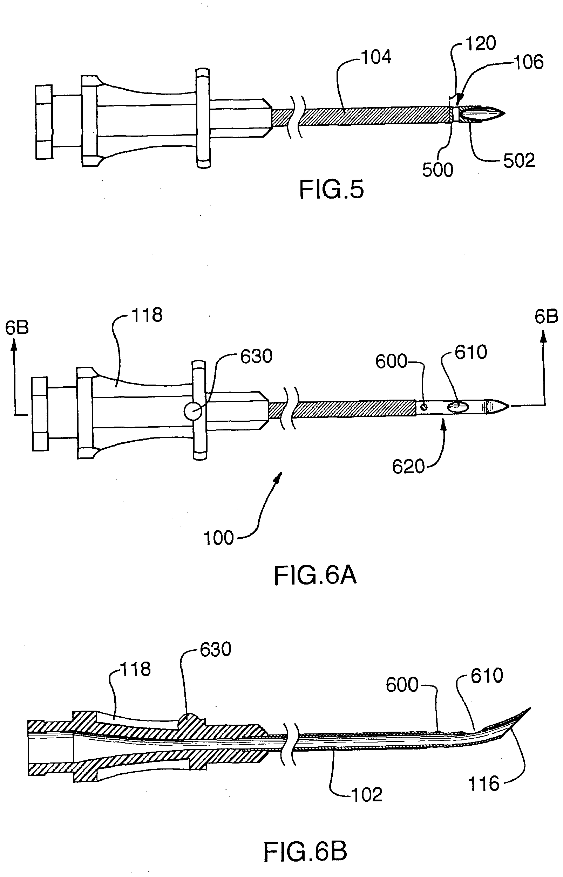

Shaft 102 defines a lumen 110 extending longitudinally from a proximal region 112 to a distal region 114 of cannula 100. In this first embodiment, distal tip portion 106 defines an aperture 116 in communication with lumen 110 of shaft 102. The combination of lumen 110 and aperture 116 allows for the introduction of a fluid or other material into a patient's body. Cannula 100 optionally further comprises a hub 118 located at or adjacent to proximal region 112. Hub 118 may be manufactured from ABS (Acrylonitrile Butadiene Styrene) or a similar material and may be attached to shaft 102 using various methods, including but not limited to insert molding, gluing and other forms of bonding. In the context of the present invention, the term hub indicates a fitting or any other means of facilitating a secure connection between separate components such as a cannula and a probe. As such, hub 118 is optionally structured to cooperatively engage and mate with a probe, stylet or other device which may be introduced into lumen 110. Hub 118 may also be useful as a handle to grasp and manipulate cannula 100. In those embodiments that comprise a hub, lumen 110 may be sized to simultaneously accommodate a stylet, probe or other device as well as a diagnostic or therapeutic agent. The diagnostic or therapeutic agents may include, but are not limited to, contrast or other chemical agents, pharmaceuticals, and biological agents. In other embodiments, lumen 110 may be designed to receive a probe, stylet or other device without having sufficient space to accommodate a diagnostic or therapeutic agent. In such embodiments, the probe, stylet or other device may be removed from lumen 110, allowing for injection of a diagnostic or therapeutic agent if so desired. It should be noted that, while cannula 100 has been described as having a single lumen, alternate embodiments with more than one lumen are also envisioned. Likewise, although this embodiment depicts a cannula with a single aperture, more than one aperture may be disposed along the cannula and the one or more apertures may be disposed at various locations along cannula 100 and are not limited to the locations shown in the appended drawings. Examples of such arrangements are described in Figures 6 - 9 below.

In the first embodiment shown in Figure 1, radiopaque band 108 is located adjacent a distal edge 120 of electrically insulating material 104, thus allowing a user to distinguish active tip 106 from the remainder of shaft 102 using radiographic imaging techniques.

The radiopaque band may completely circumscribe shaft 102, as shown in the embodiment of Figure 1, or it may only partially circumscribe shaft 102. For example, in some embodiments, the radiopaque band may only traverse 180° of the circumference of shaft 102. In such embodiments, the radiopaque band may be located on the same side of cannula 100 as the opening of aperture 116 or on the opposite side, thus allowing a user to more precisely determine the location of the opening of aperture 116.

Suitable materials for radiopaque band 108 include, but are not limited to, high-density metals such as platinum, iridium, gold, silver, tantalum or their alloys, or radiopaque polymeric compounds. Such materials are highly visible under fluoroscopic imaging and are therefore visible even at minimal thicknesses. In the embodiment depicted in Figure 1, radiopaque band 108 has the same outer diameter as electrically insulating material 104, in order to avoid increasing the force required to insert the cannula into a patient's body.

Radiopaque band 108 may be laser welded to shaft 102, thus improving the heat resistance of the band-to-cannula bond and allowing the cannula to withstand multiple thermal cycles, as may be experienced when using cannula 100 in conjunction with electrosurgical procedures, for example as described below. In addition, the laser welding technique permits a smooth taper to be formed from shaft 102 to radiopaque band 108 as shown in Figure 1. Radiopaque band 108 is tapered at least at a distal end of radiopaque band 108, although it may additionally be tapered at a proximal end as well. Providing a radiopaque marker with a tapered distal end helps reduce the force required to insert the cannula comprising the radiopaque marker into a patient's body, due to the smooth transition, provided by the tapered marker, from an outer diameter of active tip 106 to an outer diameter of insulating material 104. As mentioned above, in accordance with this first embodiment, the outer diameter of radiopaque band 108 may be substantially equivalent to the outer diameter of insulating material 104 in order to provide this smooth transition.

In alternate embodiments, radiopaque band 108 may be applied using a number of techniques known in the art, including but not limited to vapor deposition, ion implantation, dip coating, metal plating, welding, soldering and electro plating. In addition, in embodiments wherein radiopaque band 108 is manufactured from a material such as platinum iridium, the band may be fused onto shaft 102. In some embodiments, radiopaque band 108 may have a width of about 1 to about 2 mm (approximately 0.04 to 0.08 inches) and, more specifically, about 1.2 to about 1.3 mm (approximately 0.45-0.55 inches); however, this invention may be practiced with radiopaque bands of various widths and is not limited to the specific widths described in conjunction with this first embodiment.

In some embodiments, cannula 100 may be manufactured out of any of a number of conductive materials including but not limited to stainless steel, titanium, a nickel-titanium alloy or other conductive, biocompatible materials able to impart varying degrees of flexibility and strength to cannula 100. In these embodiments, cannula 100 may be overlain with one or more layers of electrically insulating material 104, defining an insulated region 122. Suitable electrically insulating materials 104 may include, but are not limited to, parylene and PTFE. In other embodiments, cannula 100 may be made from a non-conductive material such as, but not limited to PTFE, polyvinylchloride (PVC), or polyurethane, with a conductive material applied overtop of the distal tip to form active tip 106. Cannula 100 may be about 18 to about 22 AWG and about 5 to about 15 cm (approximately 2-4 inches) in length and active tip 106 may be about 2 to about 10 mm (approximately 0.075-0.4 inches) in length. However, cannula 100, as well as the active tip, may be designed in a variety of gauges and lengths and the invention is not limited in this regard.

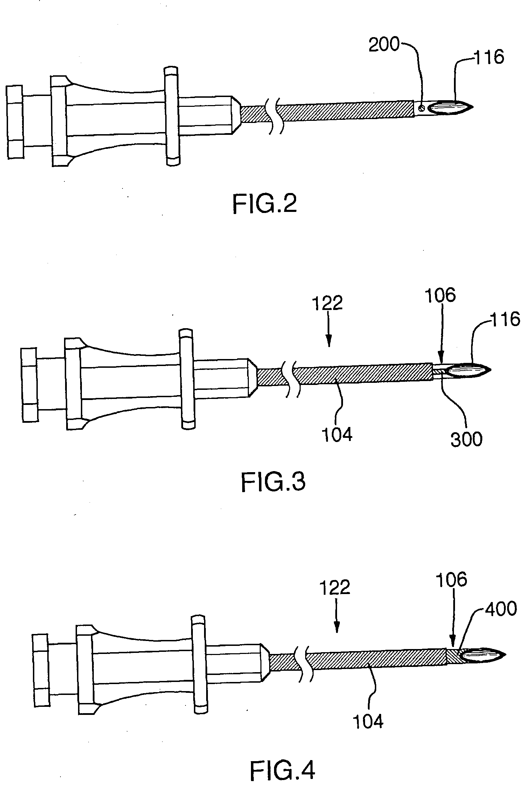

In addition to the band shown in Figures 1A and 1B, radiopaque markers of various shapes and patterns may be applied on selected portions of cannula 100 by, for example, the use of various masking techniques. Selecting specific patterns of radiopacity will allow one or more of the orientation and position of cannula 100, the position of distal tip portion 106 and the position of aperture 116 to be discerned by inspection of the fluoroscopic image. Figures 2-4 illustrate various exemplary patterns of radiopacity that are not in accordance with this invention, while Figure 5 illustrates a pattern in accordance with the invention.

Referring now to Figure 2, an alternate arrangement not in accordance with the present invention is shown. In this arrangement, a radiopaque marker 200 may take the shape of a circle and may be placed on only one side of the shaft. As discussed above, having a radiopaque marker on only one side of the shaft may be useful in distinguishing the position and orientation of aperture 116. Similarly, the direction of a beveled tip may be identified by placing a radiopaque marker on one side of the shaft. Although Figure 2 depicts a circular marker, it should be readily apparent that various other shapes and patterns, of various sizes, may be used. Figure 3 shows an alternate arrangement not in accordance with the present invention, wherein a radiopaque marker 300 runs substantially parallel to the long axis of the shaft between insulated region 122 and aperture 116 along active tip 106 thereby more precisely indicating the location of aperture 116 as well as the entire length of active tip 106. Variations of this arrangement may include various lengths and widths of radiopaque marker 300. For example, in some arrangements, radiopaque marker 300 may not terminate at an edge of insulated region 122 but may rather continue under or on top of insulating material 104. Alternatively, radiopaque marker 300 may not extend from aperture 116 to the edge of insulated region 122 but may rather extend from aperture 116 to another location along active tip 106.

Figure 4 illustrates yet another arrangement not in accordance with the present invention wherein a radiopaque marker 400 covers the entire active tip 106 of the cannula. Such an arrangement may be manufactured, for example, by masking insulated region 122 and coating distal tip portion 106 with a suitable radiopaque material, using techniques mentioned above such as vapour deposition and ion bombardment. This arrangement would render the entire active tip 106 readily distinguishable from insulated region 122 under fluoroscopic imaging. In alternate arrangements, a section of distal tip portion 106 may be left uncoated. In an additional arrangement of a radiopaque cannula, additionally radiopacity may be imparted to insulated region 122. For example, insulating material 104 may be rendered radiopaque using a number of techniques, including but not limited to vapour deposition, ion-bombardment and ion-implantation. Alternatively, a radiopaque marker may be applied onto a portion of shaft 102 along insulated region 122 prior to the application of insulating material 104. These arrangements would allow insulated region 122 to be distinguishable from active tip 106 under fluoroscopy due to its increased radiopacity.

Referring now to the embodiment in accordance with the invention shown in Figure 5, the cannula may comprise two radiopaque markers 500 and 502, located adjacent distal edge 120 of insulating material 104 and along a portion of distal tip portion 106, respectively. Such embodiments may be useful to distinguish various regions, for example a distal region and a proximal region, of active tip 106. The specific embodiment shown in Figure 5 would provide a visual frame for active tip 106, whereby a user would have precise information regarding the entire length of active tip 106 without having to increase the radiopacity of the entire active tip region. Alternatively, the cannula may comprise more than two radiopaque markers.

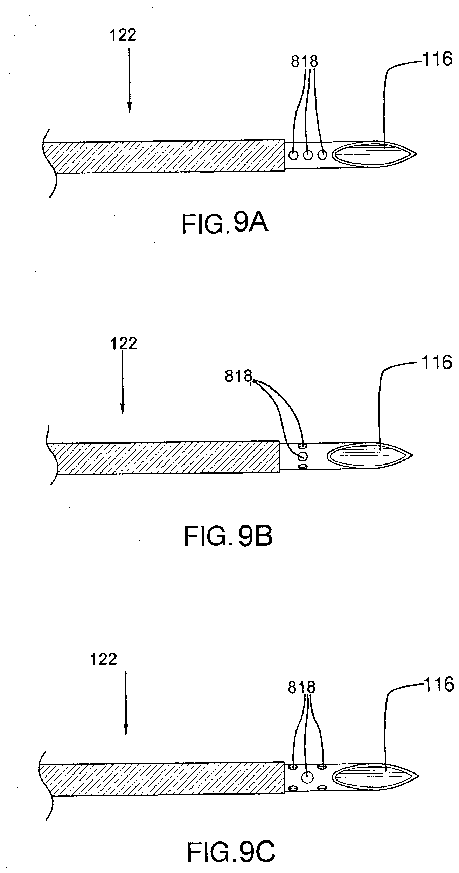

In the arrangement shown in Figures 6A and 6B, cannula 100 comprises a curved or rigidly bent distal tip portion 620 as well as a lateral aperture 610 which may serve as a delivery port for diagnostic or therapeutic agents. The term 'bent' may be understood to mean having a deviation from a longitudinal axis. This may take the form of a rigid bend or a more subtle curve, with various angles of curvature. In addition, for the purposes of this disclosure, the term 'lateral aperture' refers to an aperture, opening or hole defined through a lateral or radial surface, for example a wall, of cannula 100. Lateral aperture 610 operates to induce diagnostic or therapeutic agents introduced into cannula 100 to flow along distal tip portion 620, i.e. parallel and radial to cannula 100, effectively concentrating the diagnostic or therapeutic agents in an area to be treated by cannula 100. Lateral aperture 610 may be disposed on any location of shaft 102, including but not limited to the inner or outer surfaces of the bend of distal tip portion 620, and additional lateral apertures may be present anywhere along the lateral surface of shaft 102 as shown, for example, in Figure 9. In addition, some arrangements may further comprise an aperture 116, located at an end of distal tip portion 620 to maximize delivery of the agent to the target site.

Although the arrangement illustrated in Figures 6A and 6B shows a cannula with a single lumen, alternate arrangements may comprise a cannula with more than one lumen, whereby each lumen may be connected to one or more apertures. A radiopaque marker 600 may be located on shaft 102 in order to indicate one or more of the position and orientation of any of the lateral apertures 610 or aperture 116. The specific form and location of radiopaque marker 600 may vary and may, for example, resemble any of the aforementioned arrangements or embodiments and their equivalents. This particular arrangement of the device, comprising a lateral aperture, is especially useful for targeted delivery of anaesthetic to a nerve selected for lesioning or other treatment. In addition, having a bent tip may allow for easier manoeuvrability of the cannula through a patient's body. Although the arrangement shown in Figures 6A and 6B comprises a bent tip, cannulae with straight tips or varying degrees of curvature may also be used in conjunction with one or more lateral apertures as described in more detail below.

In addition, hub 118 may comprise a marker 630 which may be, for example, a visual, tactile, or radiopaque indicator to enable more accurate positioning of cannula 100. Marker 630 may be located, for example, on the same side of cannula 100 as a lateral aperture 610, such that it is aligned with lateral aperture 610, or on the opposite side, thus enabling a user to determine the location and/or orientation of a lateral aperture 610 after cannula 100 has been inserted into a patient's body.

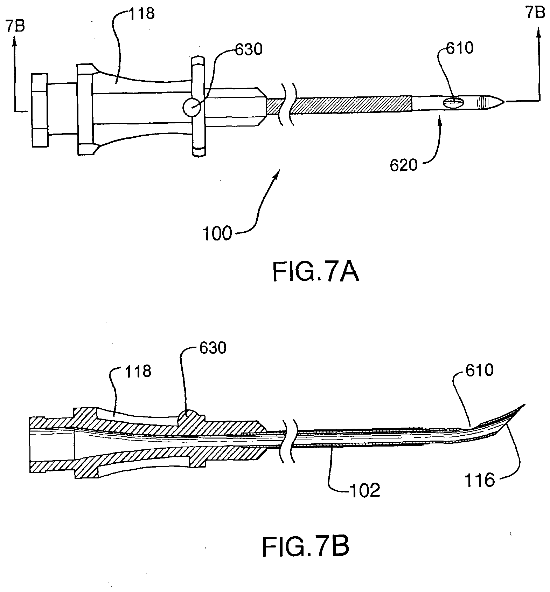

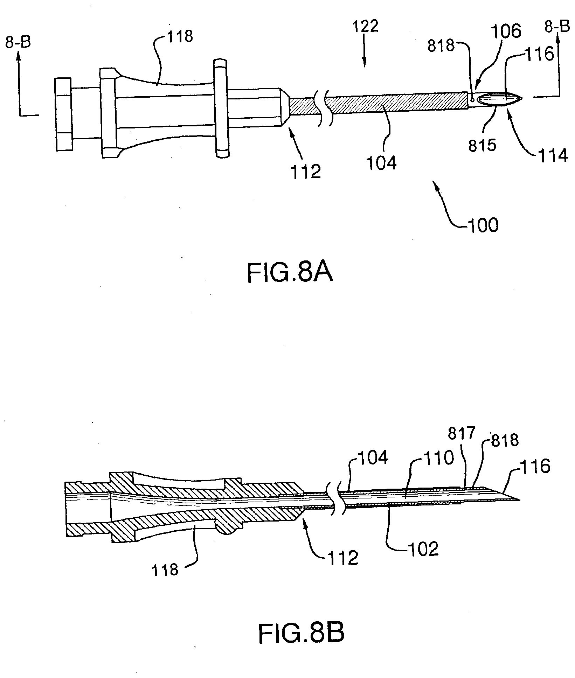

As mentioned above, in some arrangements not in accordance with the present invention, cannula 100 may not have a radiopaque marker. For example, in the arrangement shown in Figures 7A and 7B, cannula 100 comprises a curved or rigidly bent distal tip portion 620 as well as a lateral aperture 610 as shown in Figure 6. Figures 8A and 8B show a further arrangement of a cannula 100 not in accordance with the present invention comprising a lateral aperture 818. Figure 8A shows a top plan view of cannula 100 while Figure 8B illustrates a sectional view of cannula 100, taken along the line 8B in Figure 8A. As mentioned above with respect to Figure 1, elongate member 102 may be a straight shaft 102 comprising a proximal region 112 and a distal region 114. Distal region 114 comprises a distal tip 106 which may be electrically exposed and conductive while the outer surface of cannula 100, between distal tip 106 and proximal region 112, may be non-conductive. Shaft 102 defines a lumen 110 extending longitudinally between proximal region 112 and distal region 114 of cannula 100. In this embodiment, a distal end 815 of shaft 102 defines at least one distal aperture 116 while a wall 817 of shaft 102 defines at least one lateral aperture 818 therethrough. In this arrangement, distal aperture 116 and lateral aperture 818 are both in communication with lumen 110 of shaft 102.

As described above with respect to Figure 6, a cannula 100 as shown in the arrangement of Figure 8 may allow for distribution of a treatment composition, such as diagnostic or therapeutic fluid agents through both lateral aperture 818 and distal aperture 116. Lateral aperture 818 may facilitate delivery to a larger volume of tissue, and may also induce the fluid agents to flow along the length of distal tip 106, parallel to cannula 100, which may effectively concentrate the diagnostic or therapeutic agents in an area to be treated. In other words, a treatment composition flowing through lateral aperture 818 may flow longitudinally along a surface of cannula 100 or may radiate away from cannula 100, which may not be possible using only a distal aperture 116. In this way, anesthetic, contrast fluids, or other diagnostic or therapeutic agents injected through hub 118 may be discharged through apertures 116 and 818 on a distal and lateral portion of cannula 100. Lateral aperture 818 may have a smooth or rounded wall, which may serve to minimise or reduce trauma to bodily tissue. For example, some arrangements may have a lateral aperture 818 with a smooth outer circumferential edge.

A distal aperture 116 may, alternatively or in addition, be used for the introduction or exposure of one or more treatment devices to the tissue or other material in the vicinity of cannula 100. Some treatment applications call for the use of measuring devices that directly contact the tissue being measured, in order to gain a more accurate reading. Such measuring devices, described in further detail below, may include, but are not limited to, temperature, impedance, or pressure sensors. The presence of a distal aperture 116 allows a measuring device to be inserted through cannula 100 to contact the tissue. Other devices that may be inserted through cannula 100 to access tissue adjacent distal aperture 116 may include, but are not limited to, one or more devices for removing material, one or more devices for adding material, and one or more devices for cutting, penetrating, puncturing or perforating tissue.

Various measuring devices may be used in conjunction with the cannula of the present invention to monitor physiological parameters such as temperature and pressure. Means for monitoring temperature may include, but are not limited to, thermocouples, thermistors and thermometers. Means for monitoring pressure may include, but are not limited to, pressure transducers and fluid-filled lumens in communication with fluid in a patient's body. Some embodiments of the present invention may comprise some means for monitoring electrical impedance, in order to aid in positioning the cannula within a patient's body.

As mentioned above, in alternate arrangements, lateral aperture 818 may be disposed on any lateral location of shaft 102, and additional lateral apertures 818 may be present anywhere and in any shape/configuration/arrangement along the surface of shaft 102, as may be useful for a given treatment or tissue. Various alternate arrangements of distal region 114 of cannula 100 not in accordance with the invention are illustrated in Figures 9A to 9C. In the arrangement shown in Figure 9A, cannula 100 comprises a row of lateral apertures 818 substantially parallel to a longitudinal axis of shaft 102. In another arrangement, cannula 100 may comprise numerous lateral apertures 818 in a circumferential arrangement around shaft 102, substantially perpendicular to a longitudinal axis of shaft 102, as shown in Figure 9B. Figure 9C depicts an arrangement of cannula 100 with multiple lateral apertures 818 of varying sizes disposed randomly/spaced-apart along cannula 100.

In a further arrangement, various apertures may be located at desired locations along, for example, active tip 106 in order to provide regions of electrical discontinuities, where current will not be conducted due to discontinuities in the surface of active tip 106. Providing such apertures in a desired pattern and/or distribution may serve to alter the current density along the surface of active tip 106 in a predetermined fashion. This may in turn allow for the formation of a lesion with a specified size and/or shape in a patient's body, since lesion characteristics may be related to the current density delivered from a treatment device.

Although the arrangements illustrated in Figures 7 - 9 show a cannula with a single lumen, alternate arrangements may comprise a cannula with additional lumens, whereby each lumen may be connected to one or more lateral or distal apertures. In some arrangements, for example, there may be an exclusive, one-to-one relationship between apertures and lumens. In other words, in such arrangements, each aperture may be in communication with a single lumen and there may be one lumen per aperture. In some arrangements, cannula 100 may comprise one or more structures such as, for example, hypotubes or other means for defining a lumen or channel disposed within lumen 110 of shaft 102, each hypotube defining a lumen therethrough. In such arrangements, lateral aperture 818 may be in fluid communication with a first lumen defined by, for example, shaft 102, while distal aperture 116 may be in fluid communication with a second lumen defined by, for example, a hypotube or other structure disposed within shaft 102. Other structures or means for defining a lumen may include, but are not limited to, flexible, rigid or semirigid tubing or any other substantially hollow elongate member. As the arrangements of Figures 9A and 9C indicate, the number, locations, shapes and configurations of any lateral apertures may vary depending on the specific application or arrangement.

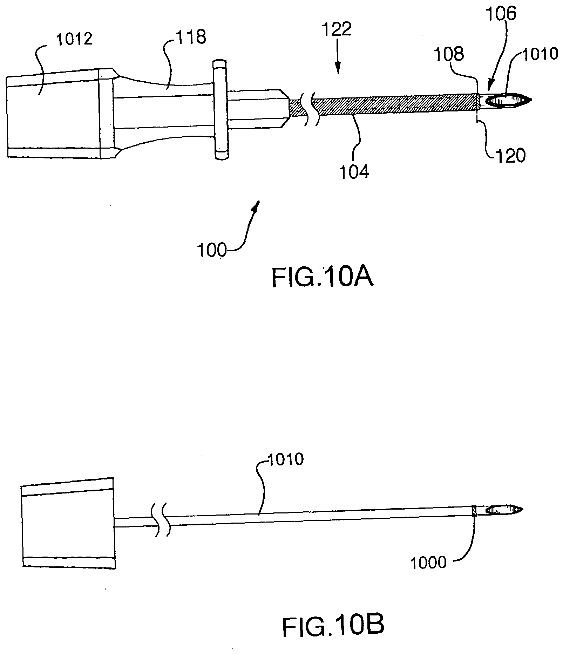

Referring now to Figure 10A, some embodiments of cannula 100 in accordance with the invention comprise a lumen into which a removable stylet 1010 or probe (not shown) may be inserted. Thus, lumen 110 may be capable of receiving at least a portion of stylet 1010. In this embodiment, stylet 1010, shown in dotted outline, is adapted to assist in piercing a patient's skin and tissue for entry to a treatment area or target site. Inserting a stylet into a cannula prior to insertion of the cannula into a patient's body helps to occlude any apertures in the cannula shaft so as to ensure that no tissue is forced into a lumen of the cannula. Stylet 1010 may comprise a cap 1012 adapted to cooperatively engage and mate with hub 118 of cannula 100. Stylet 1010 may conform to the shape of the distal end of cannula 100; for example, stylet 1010 may have a pointed tip with a trocar, conical, bevel, or other shape to allow for easy penetration of tissue when cannula 100 and stylet 1010 are introduced into the patient's body. Stylet 1010 may be temporarily or permanently attached to cannula 100, for example by welding, soldering, crimping or any other means for bonding. In such embodiments, stylet 1010 may not extend the full length of lumen 110 so that lumen 110 can further accommodate a probe. Stylet 1010 may fully or partially occlude the distal end of cannula 100. One embodiment of a kit of the present invention may comprise at least one cannula 100, at least one stylet 1010 and at least one probe. In the embodiment shown, and as described above with respect to Figure 1 , radiopaque band 108 is located adjacent a distal edge 120 of electrically insulating material 104, thereby rendering an insulated region 122 of the shaft distinguishable from active tip 106 under fluoroscopy.

Figure 10B shows a stylet 1010 comprising a radiopaque marker 1000. Radiopaque marker 1000 may be located anywhere on stylet 1010 and may adopt any shape and size, including but not limited to those described in the embodiments and arrangements of Figures 1-5 and their equivalents. In the arrangement shown in Figure 10B, radiopaque marker 1000 on stylet 1010 may serve to identify a specific portion of the cannula shaft when stylet 1010 is fully disposed within a lumen of the cannula. For example, in the arrangement shown in Figure 10B, radiopaque marker 1000 may allow active tip 106 of cannula 100 (shown in Figure 1) to be readily distinguishable from insulated region 122 under radiographic imaging when stylet 1010 is fully disposed within cannula 100. It would be beneficial, in some instances, to place a radiopaque marker on the stylet rather than on the cannula itself so that this invention may be practiced with pre-existing cannulae that lack radiopaque markers without having to incorporate such radiopaque markers onto the cannulae themselves. Furthermore, incorporating a radiopaque marker onto a stylet, rather than the cannula shaft, ensures that no additional force will be required to insert the cannula into the patient's body since the stylet is located within the cannula and the cannula itself remains unchanged.

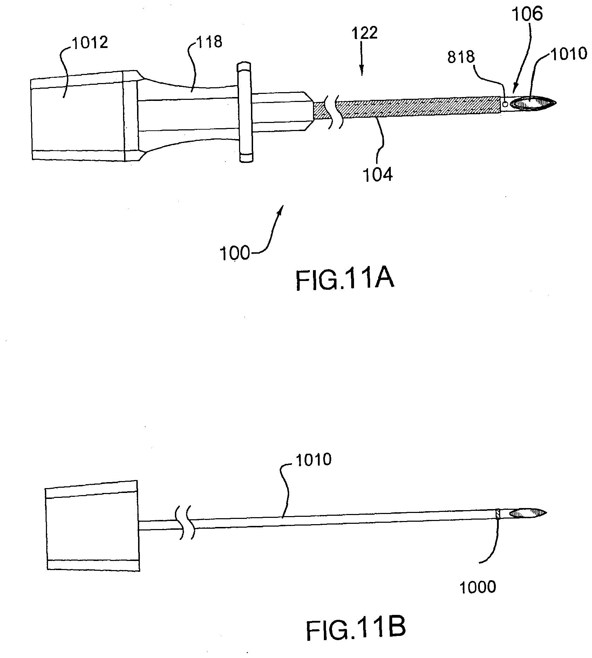

Any of the embodiments and arrangements described in Figures 1-9 may be used in conjunction with a stylet 1010 and the invention is not limited in this regard. For example, and referring now to Figures 11A and 11B, a further arrangement of cannula 100 comprises a lumen 110 sized to hold a stylet 1010 as described above, as well as at least one lateral aperture 818.

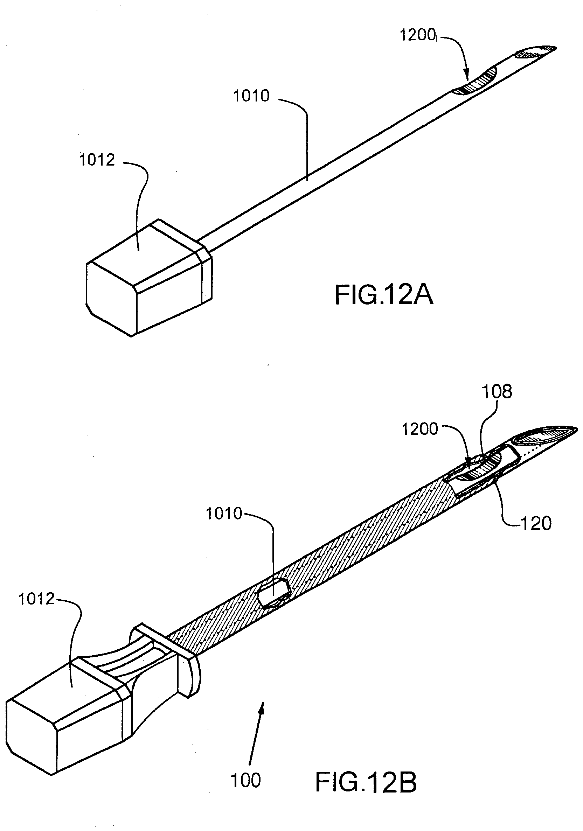

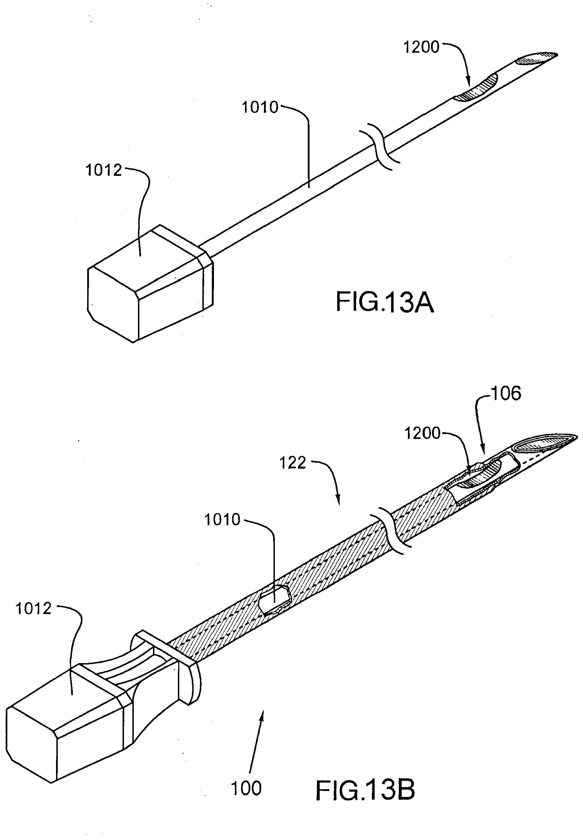

Referring now to Figures 12 and 13, the radiopacity of stylet 1010 may be reduced at a region 1200 by reducing the mass of stylet 1010 at that location. The mass of stylet 1010 may be reduced by removing material from stylet 1010 through grinding techniques or any other means for removing material. Alternatively, stylet 1010 may be originally manufactured with a reduced mass about region 1200. In the arrangement shown in Figure 12B, stylet 1010 with reduced mass at region 1200 could be used to improve the fluoroscopic visibility of radiopaque band 108 whereby region 1200 of stylet 1010 is aligned with the location of radiopaque band 108 and, due to the reduced radiopacity of region 1200 (owing to the reduced mass of stylet 1010 at that location), the visibility of radiopaque band 108 may be enhanced. In alternate arrangements not in accordance with the invention, for example as shown in Figure 13B, the cannula does not include any radiopaque markers. In such arrangements, the region of reduced mass of the stylet may be aligned with a distal edge of the insulated region of the cannula and, due to the reduced radiopacity of the stylet in this region, the distal edge of the insulated region may be more easily distinguished under fluoroscopy, thus allowing a user to determine the precise location of an active tip located adjacent the distal edge of the insulated region. In further arrangements, a radiopaque marker may be incorporated onto a stylet 1010 having a region 1200 of reduced radiopacity, whereby the radiopaque marker may be used to distinguish active tip 106 from insulated region 122, and the region 1200 of reduced radiopacity may indicate the location of a lateral aperture. The region 1200 of reduced radiopacity may, in some embodiments, be located elsewhere along stylet 1010.

In further arrangements, a radiopaque marker may be incorporated onto the stylet in conjunction with a region of reduced mass in order to allow for a thicker radiopaque marker with improved fluoroscopic visibility. In an alternative arrangement, a stylet, or a portion thereof, could be manufactured from materials of lower radiopacity than the cannula itself (for example, plastics or other non-metallic substances) in order to allow greater illumination of the radiopaque marker on the cannula under fluoroscopy. In other words, if the stylet is highly radiopaque, a radiopaque marker on the cannula will not be visible using fluoroscopy when the stylet is inserted into the cannula because the radiopacity of the stylet will mask the increased radiopacity of the radiopaque marker. Thus, if the stylet is made of a less radiopaque (or non-radiopaque) material, the radiopaque marker on the cannula may become visible even while the stylet is located within the cannula.

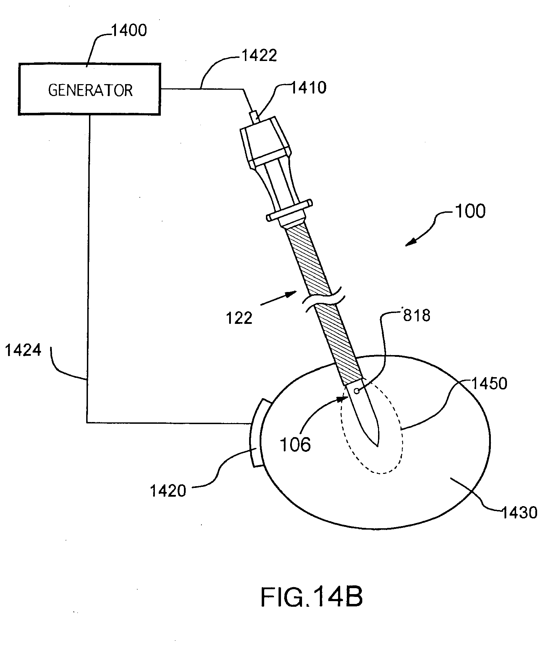

Figures 14A and 14B show alternate an embodiment and an arrangement of an electrosurgical system incorporating a cannula. Figure 14A shows an embodiment of a system incorporating a cannula of the present invention, while Figure 14B shows a system incorporating a cannula not in accordance with the invention. The electrosurgical system generally comprises an energy source 1400, a cannula 100 of the present invention, a probe 1410, a reference electrode 1420 and electrical connections 1422 and 1424. The system may further comprise a stylet, a monitoring or measuring device, a means for cooling or various other components. The means for cooling may comprise, for example, peristaltic pumps for supplying a cooling fluid to one or more of cannula 100 and probe 1410. In use, and as illustrated in Figures 14A and 14B, at least a portion of cannula 100 and probe 1410 are located in a region of a patient's body 1430, while reference electrode 1420 is placed at a location on the surface of body 1430. The components of the electrosurgical system of Figure 14 will now be described in greater detail.

Energy source 1400 may be any device capable of operating as a source of energy, for example an energy generator 1400. In one embodiment, energy generator 1400 is an electrical generator capable of providing high-frequency electrical current. Specifically, energy generator 1400 may be operable in a radio-frequency (RF) range, for example, from about 260 kHz to about 1.5 MHz, and may be capable of delivering sufficient power so as to effectively treat a patient's pain. As will be discussed below, treatment of pain may involve the creation of a lesion in a specific neural tissue through heat generated by the application of RF energy. In some embodiments, energy generator 1400 may be operable in a range of about 300 kHz to about 600 kHz.

Reference electrode 1420 may be sufficiently large to prevent localized heating on the surface of body 1430 where reference electrode 1420 is placed. In some embodiments, more than one reference electrode may be provided. In alternate embodiments, probe 1410 may contain two or more separate electrodes, whereby at least one electrode (the active electrode) may be electrically coupled to cannula 100 and at least one additional electrode may act as a reference electrode. In additional embodiments, reference electrode 1420 may be replaced by one or more reference electrodes located on one or more additional probes inserted into the body proximate probe 1410. In yet further embodiments, the system may deliver energy in a bipolar configuration, as described below.

Electrical connections 1422 and 1424 may be any means of conveying or transmitting energy from generator 1400 to probe 1410 and from reference electrode 1420 to generator 1400. For example, electrical connections 1422 and 1424 may comprise electrical cables and/or wires along with associated connectors for interfacing with generator 1400, probe 1410 and reference electrode 1420. Various other means of electrical coupling are possible and the invention is not limited in this regard.

In some embodiments, cannula 100 may be structured so that an electrical connection between probe 1410 and cannula 100 is established by the physical apposition of these elements. For example, if cannula 100 is manufactured from a conductive material, probe 1410 may be inserted within cannula 100 such that physical contact between cannula 100 and probe 1410 may be sufficient to allow for a transfer of electrical energy from the probe to the cannula. In further embodiments, other means for transferring energy from probe 1410 to cannula 100 may be utilized. In some embodiments, probe 1410 may be cooled, for example, by having one or more internal lumens for the circulation of cooling fluid, or by thermoelectric cooling. In the embodiment shown in Figure 14A, which is in accordance with the invention, cannula 100 comprises a radiopaque marker 108 adapted to allow a user to distinguish an electrically insulated region 122 from an active tip 106 using fluoroscopic imaging, as has been described throughout this specification. In the arrangement of Figure 14B, cannula 100 comprises a lateral aperture 818 to allow for more focused delivery of a treatment composition, as described above.

In general, high frequency electrical current flows from generator 1400 via electrical connection 1422 to probe 1410 and via probe 1410 to active tip 106. This delivery of energy results in electrical stimulation or high frequency heating of tissue in the region surrounding active tip 106. If the tissue surrounding active tip 106 comprises one or more neural structures, the formation of a lesion 1450 may lead to pain relief due to the denervation of said neural structures.

Embodiments of the present invention comprising one or more radiopaque markers are particularly useful for procedures where precise knowledge of the area to be treated is critical. In addition, embodiments comprising one or more lateral apertures may be particularly useful in treatment procedures wherein a target structure, for example a target nerve, lies substantially parallel to the cannula. In such a procedure, a lateral aperture, as described above, may be used to deliver a treatment composition, such as an anesthetic, to the target structure more precisely. In facet joint denervation, for example, it is critical that certain nerves, specifically those of the sympathetic chain, are not damaged during the treatment procedure. The sympathetic chain refers to either of the pair of ganglionated longitudinal cords of the sympathetic nervous system of which one is situated on each side of the spinal column. Furthermore, the target nerve in such procedures may be located substantially parallel to the device used for delivering energy to the target nerve. Thus, embodiments of the present invention may be beneficially utilized in the performance of this, as well as other, procedures. An exemplary radio frequency treatment procedure, using a device of the present invention, may be generally performed as follows: inserting a cannula into the body adjacent the target treatment site; delivering energy to the treatment site via the cannula; and delivering a treatment composition to the treatment site via at least one lateral aperture before, after or during the delivery of energy. The delivery of energy may be useful in treating the patient's pain.

A more detailed exemplary method may proceed as follows: with a patient lying prone, a cannula containing a stylet disposed within a lumen of the cannula, is inserted and positioned parallel to the target treatment site to be lesioned using visualization means such as fluoroscopic guidance; a radiopaque or radiolucent (for example, another visual or tactile marker) marker that may be located on one or more of the cannula and the stylet may provide improved visualization to assist in positioning the cannula. Once positioned, the stylet is removed and replaced by an electrosurgical probe which may have a protruding distal thermocouple, such that the thermocouple extends in part through the distal aperture of the cannula. Following a test for motor or sensory stimulation, as an added safety measure, an anaesthetic fluid or other agents may be delivered to the vicinity of the treatment site via one or more lateral apertures and/or a distal aperture. In such cases, a radiopaque marker may be useful in determining the location of any apertures present on the cannula in order to effectively direct the injection of any such agents to the appropriate location. Next, energy is delivered from an energy generator through the probe to the active tip of the cannula in order to create a lesion adjacent the active tip. Energy may be delivered, for example, at a frequency of about 300 kHz to about 600 kHz in order to create a lesion. During energy delivery, the temperature of the tissue adjacent to or in the vicinity of the distal tip of the cannula may be monitored by the thermocouple protruding from the electrosurgical probe.

In alternate exemplary arrangements, a method may comprise a number of variations to the aforementioned method, may omit one or more steps or may add one or more additional steps. The positioning of the patient and the depth and angle of insertion of the cannula may depend on a number of factors, including the location and tissue type at the target treatment site, and on the nature of the procedure(s) to be performed. The method also provides for the insertion of multiple cannulae of the present invention over the course of a treatment procedure, whereby any or all of the cannulae may be positioned under fluoroscopic guidance, as described above. During and/or following insertion, a number of means for visualizing may be used, including, but not limited to fluorescence, MRI, X-ray, and laparoscopic imaging, and these means for visualizing may or may not be aided by the use of markers such as, but not limited to, radiopaque markers, radiolucent markers, tactile markers, and visual markers, for example, on the proximal portions of the cannula. The step of inserting the cannula into the body may additionally comprise a step of positioning the cannula within the body. This positioning step may involve guiding or steering a part of the cannula using a means for manipulating the cannula, by, for example, actuating a change in the shape of at least a portion of the cannula; twisting, turning, pushing, pulling, expanding, or contracting at least a portion of the cannula; or extending or retracting at least a portion of the cannula. Following insertion, a stylet may be removed, if present, though the method may be performed using cannulae with no stylet or other means for occluding. Alternatively, a stylet may remain within the cannula.

Prior to delivery of energy, one or more additional treatment devices may be inserted and one or more additional treatment procedures may be performed on the tissue. Additional procedures may include, but are not limited to: removal of material, addition of material (including therapeutic fluid agents, such as anaesthetic), and application of cooling. For example, a step comprising the addition of material may comprise the delivery of a treatment composition such as a diagnostic or therapeutic agent including, but not limited to: anesthetic, cooling fluids and chemical, pharmaceutical, and biological agents. Chemical agents may include non-pharmaceutical chemicals, such as ethanol, phenol, chelating agents, tissue sealants, cryogenic fluids, and contrast agents for imaging particular structures of the body, including contrast agents for X-ray, fluoroscopy, ultrasound, computerized tomography (CT), and MRI. Pharmaceutical agents may include drugs commonly available to treat disease, such as pain relievers, anti-cancer agents, antibiotics, antithrombotic agents, anti-virals, and enzyme inhibitors. Biological agents may include nucleic acids, amino acids, cells, viruses, prions, biochemicals, vitamins, and hormones. Cooling may be supplied by means other than by delivery of a cooling fluid through an aperture, for example by circulation of a cooling fluid through a closed lumen, or by the use of thermoelectric cooling. In addition, conductive fluids may be delivered to a treatment site in order to allow for the creation of a larger lesion at the treatment site. Alternatively or in addition, delivery of fluid may be used to create a lesion of a desired shape and/or orientation. Material may be added through one or more lateral apertures and/or one or more distal apertures.

The exemplary method may also comprise, in some examples, utilizing the distal aperture in the performance of a secondary procedure. For example, the method may include the insertion of additional treatment devices such that they access the tissue surrounding the cannula through the distal aperture. For example, a device may be inserted through the distal aperture such that at least a portion of the device is in contact with tissue. In some examples, the method may comprise the steps of inserting a measuring device into the cannula and using said measuring device to measure a property of the treatment site or of any component of the cannula. Measuring devices may be used to measure, for example, temperature, pressure, or impedance or other physiological parameters. In some examples, measuring devices may be operable to directly measure a property of the tissue of the treatment site by contacting said tissue through the distal aperture of the cannula.

The step of inserting a probe into the cannula describes the insertion of any elongated device capable of delivering energy, as described above. Systems used in conjunction with the current method may additionally comprise means for cooling, measurement devices or additional functional elements for performing treatments. In one example, the probe and generator are configured to be operable to deliver stimulation energy to the treatment site, and a measuring device (integral or external to the probe) may be used to measure the response of neural or muscular tissue to said stimulation energy; in this example, the method may comprise the additional steps of delivering energy at a stimulation frequency (for example, about 1 to about 100 Hz) and detecting a response to said energy. Additional functional elements, which may be able to be used to perform secondary procedures may include, but are not limited to elements for removing material from or adding material to the treatment site. Examples of functional elements for removing material include, but are not limited to, clamps, knives, jaws, augers, forceps, scissors, and suction devices. Examples of materials that may be added to a treatment site include, but are not limited to therapeutic agents as described above, sealants, structural or supporting material (synthetic or biological), and materials used to aid in tracing or visualization. Thus, a secondary procedure may involve one or more of adding material to a treatment site and removing material from a treatment site. Cooling means, measuring devices, and additional functional elements may be used prior to, during, or following the step of delivering energy for treatment. Furthermore, in some examples, at least a part of the probe may contact the tissue of the treatment site through the distal aperture.

The step of delivering energy may involve delivering energy in a bipolar configuration between two or more cannulae located at spaced apart sites within the body, or delivering energy in a monopolar configuration between one or more cannulae and a reference electrode at a remote location on or in the body. Alternatively, energy may be delivered in various other multipolar or multiphasic configurations. Energy may be delivered continuously, or may be interrupted, for example according to a pre-determined duty cycle. Delivery of energy is an interrupted or discontinuous manner may be referred to as 'pulsed' energy delivery.

The step of delivering energy may also be performed, in some examples, in conjunction with a step of automatically or manually modifying a treatment procedure (for example, modifying energy delivery) in response to one or more measured properties or parameters. These measured parameters may include, but are not limited to, temperature, position of the probe(s) or impedance. For example, if a temperature measurement is determined to be outside of a desired range, a treatment procedure may be modified by, for example, altering the amount of energy delivered, modifying or modulating a cooling means in some way, or terminating the procedure. In such examples, a feedback system may be associated with or incorporated into the energy source so that any modification of a treatment procedure in response to a measured parameter may occur automatically, without any input from a user. Such a feedback system may include, for example, one or more of a processor and a controller. In other examples, there may not be an automatic feedback apparatus in place, in which case a user may manually modify a treatment procedure in response to a measured parameter. In addition to modifying a treatment procedure based on measured parameters, a step of determining the initial parameters may be used in a treatment procedure (for example, the initial maximum power level or tissue temperature, temperature ramp rate, etc.) using information that is known about the particular tissue to be treated. For example, if the tissue to be treated is a patient's sacrum, and if pre- treatment testing reveals specific information about the sacrum (this information may include, but is not limited to: the topology of the sacrum, location of specific nerves, etc.), that information may be used to decide on what parameters to use initially for the treatment procedure.

Examples of a treatment procedure as described herein may be useful in order to treat a patient's pain. By creating a lesion in a region of tissue comprising one or more neural structures, the transmission of pain may be blocked. With respect to back pain in particular, such treatment procedures may be applied to several tissues, including intervertebral discs, facet joints, and sacroiliac joints as well as the vertebrae themselves (in a process known as intraosseous denervation). In addition to treating neural structures, the application of RF energy has been effectively used to treat tumors and cardiac tissue, among others. The cannula 100 may comprise other useful features in addition to those mentioned above. For example, a cannula 100 of the present invention may comprise pressure monitoring sensors such as, for example, pressure transducers and fluid-filled lumens in communication with fluid in a patient's body. In addition, some cannulas may comprise some means of monitoring electrical impedance, in order to aid in positioning the cannula within a patient's body. Furthermore, in some or all of the embodiments described in this specification, distal tip portion 106 of cannula 100 may be manufactured as a separate piece and may then be attached by some means to shaft 102.

It should be noted that the terms radiopaque band, marker, marking etc. as used herein denote any addition or reduction of material that increases or reduces the radiopacity of the device. Furthermore, the terms probe, cannula, stylet etc. are not intended to be limiting and denote any medical and surgical tools that can be used to perform similar functions to those described. In addition, the invention is not limited to be used in the clinical applications disclosed herein, and other procedures wherein a device of the present invention would be useful are envisaged. Furthermore, the cannulae described in the present invention are not intended to be limited to a specific length or gauge, as has been mentioned.

The embodiments of the invention described above are intended to be exemplary only. The scope of the invention is therefore intended to be limited solely by the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims. Citation or identification of any reference in this application shall not be construed as an admission that such reference is available as prior art to the present invention.

## Claims

1. A cannula (100) for insertion into a patient's body, the cannula comprising:
an elongate member (102) having a proximal region and a distal region (106) and defining a lumen (110) therebetween;
a radiopaque marker (108) associated with the elongate member for identifying a portion of said elongate member under radiographic imaging;
wherein said elongate member (102) is electrically conductive, said elongate member comprising:
an electrically insulated region (104);
an electrically exposed region (106);
wherein said radiopaque marker (108) is associated with at least one of said electrically insulated region (104) and said electrically exposed region (106) and is positioned for distinguishing the exposed region from the insulated region under radiographic imaging, the radiopaque marker (108) being located adjacent a distal edge (120) of the insulated region:
**characterised in** said radiopaque marker (108) being shaped so as to reduce a force required to insert said elongate member into said patient's body;
and wherein said radiopaque marker (108) comprises a tapered distal end, the tapered radiopaque marker providing a smooth transition from an outer diameter of the electrically exposed region to an outer diameter of the electrically insulated region (104).

2. The cannula of claim 1, wherein a distal end of said cannula (100) defines an aperture (116) in communication with said lumen.

3. The cannula of claim 1 or 2, wherein said cannula is operable to be connected to an energy source.

4. The cannula of any one of claims 1 to 3, further comprising a stylet, said stylet being insertable into said lumen (110).

5. The cannula of any preceding claim, wherein the elongate member (102) is coated with an electrically insulated material (104) to provide the electrically insulated region.

6. The cannula of any preceding claim, wherein the radiopaque marker is laser welded to the elongate member.

7. The cannula of any preceding claim, wherein the radiopaque marker is a radiopaque band.

8. The cannula of any preceding claim, wherein the radiopaque marker is additionally tapered at the proximal end.

9. The cannula of any preceding claim, wherein the outer diameter of the radiopaque marker is substantially equivalent to the outer diameter of the insulated region.

10. A system for delivering energy to a patient's body, said system comprising:
an energy source (1400)
a cannula in accordance with claim 1;
means for delivering energy from said energy source (1400) to said cannula; and
a reference electrode (1420).

## Patentansprüche

1. Kanüle (100) zum Einführen in den Körper eines Patienten, umfassend:
ein längliches Element (102), das einen proximalen Bereich und einen distalen Bereich (106) besitzt, und ein Lumen (110) dazwischen definiert;
einen röntgendichten Marker (108), der mit dem länglichen Element verbunden ist, um unter radiographischer Bildgebung einen Abschnitt des länglichen Elements zu identifizieren;
wobei das längliche Element (102) elektrisch leitfähig ist, wobei das längliche Element umfasst:
einen elektrisch isolierten Bereich (104);
einen elektrisch ausgesetzten Bereich (106);
wobei der röntgendichte Marker (108) mit mindestens einem des elektrisch isolierten Bereichs (104) und des elektrisch ausgesetzten Bereichs (106) verbunden ist und zur Unterscheidung des ausgesetzten Bereichs von dem isolierten Bereich unter radiographischer Bildgebung positioniert ist, wobei der röntgendichte Marker (108) benachbart zu einem distalen Rand (120) des isolierten Bereichs angeordnet ist:
**dadurch gekennzeichnet, dass** der röntgendichte Marker (108) so geformt ist, dass eine Kraft reduziert wird, die erforderlich ist, um das längliche Element in den Körper des Patienten einzuführen;
und wobei der röntgendichte Marker (108) ein zugespitztes distales Ende umfasst, wobei der zugespitzte röntgendichte Marker einen glatten Übergang von einem Außendurchmesser des elektrisch ausgesetzten Bereichs zu einem Außendurchmesser des elektrisch isolierten Bereichs (104) bereitstellt.

2. Kanüle nach Anspruch 1, wobei ein distales Ende der Kanüle (100) eine Öffnung (116) definiert, die mit dem Lumen in Verbindung steht.

3. Kanüle aus Anspruch 1 oder 2, wobei die Kanüle eingerichtet ist, um an eine Energiequelle angeschlossen zu werden.

4. Kanüle nach einem der Ansprüche 1 bis 3, weiter umfassend eine Sonde, die in das Lumen (110) einführbar ist.

5. Kanüle nach einem der vorstehenden Ansprüche, wobei das längliche Element (102) mit einem elektrisch isolierten Material (104) beschichtet ist, um den elektrisch isolierten Bereich bereitzustellen.

6. Kanüle nach einem der vorstehenden Ansprüche, wobei der röntgendichte Marker mit dem länglichen Element laserverschweißt ist.

7. Kanüle nach einem der vorstehenden Ansprüche, wobei der röntgendichte Marker ein röntgendichtes Band ist.

8. Kanüle nach einem der vorstehenden Ansprüche, wobei der röntgendichte Marker zusätzlich am proximalen Ende zugespitzt ist.

9. Kanüle nach einem der vorstehenden Ansprüche, wobei der Außendurchmesser des röntgendichten Markers im Wesentlichen dem Außendurchmesser des isolierten Bereiches entspricht.

10. System, das Energie an den Körper eines Patienten liefert, umfassend:
eine Energiequelle (1400)
eine Kanüle nach Anspruch 1;
Mittel zum Liefern von Energie aus der besagten Energiequelle (1400) an die Kanüle;
und
eine Referenzelektrode (1420).

## Revendications

1. Canule (100) pour une insertion dans le corps d'un patient, la canule comprenant :
un organe allongé (102) ayant une zone proximale et une zone distale (106) et définissant une lumière (110) entre celles-ci ;
un marqueur radio-opaque (108) associé à l'organe allongé pour identifier une partie dudit organe allongé sous imagerie radiographique ;
dans laquelle ledit organe allongé (102) est électriquement conducteur, ledit organe allongé comprenant :
une zone électriquement isolée (104) ;
une zone électriquement exposée (106) ;
dans laquelle ledit marqueur radio-opaque (108) est associé à au moins une parmi ladite zone électriquement isolée (104) et ladite zone électriquement exposée (106) et est positionné pour distinguer la zone exposée de la zone isolée sous imagerie radiographique, le marqueur radio-opaque (108) étant positionné au voisinage d'un bord distal (120) de la zone isolée ;
**caractérisée en ce que** ledit marqueur radio-opaque (108) ayant une forme pour réduire une force requise pour insérer ledit organe allongé dans le corps dudit patient ;
et dans laquelle ledit marqueur radio-opaque (108) comprend une extrémité distale pointue, le marqueur radio-opaque pointu fournissant une transition lisse d'un diamètre extérieur de la zone électriquement exposée à un diamètre extérieur de la zone électriquement isolée (104) .

2. Canule selon la revendication 1, dans laquelle une extrémité distale de ladite canule (100) définit une ouverture (116) en communication avec ladite lumière.

3. Canule selon la revendication 1 ou 2, dans lequel ladite canule est utilisable pour être raccordée à une source d'énergie.

4. Canule selon l'une quelconque des revendications 1 à 3, comprenant en outre un stylet, ledit stylet étant insérable dans ladite lumière (110).

5. Canule selon l'une quelconque des revendications précédentes, dans laquelle l'organe allongé (102) est revêtu d'un matériau électriquement isolé (104) pour fournir la zone électriquement isolée.

6. Canule selon l'une quelconque des revendications précédentes, dans laquelle le marqueur radio-opaque est soudé par laser à l'organe allongé.

7. Canule selon l'une quelconque des revendications précédentes, dans laquelle le marqueur radio-opaque est une bande radio-opaque.

8. Canule selon l'une quelconque des revendications précédentes, dans laquelle le marqueur radio-opaque est de plus pointu à l'extrémité proximale.

9. Canule selon l'une quelconque des revendications précédentes, dans laquelle le diamètre extérieur du marqueur radio-opaque est sensiblement équivalent au diamètre extérieur de la zone isolée.

10. Système pour délivrer de l'énergie au corps d'un patient, ledit système comprenant :
une source d'énergie (1400) ;
une canule selon la revendication 1 ;
des moyens pour délivrer de l'énergie de ladite source d'énergie (1400) à ladite canule ; et
une électrode de référence (1420).
